(19) **Europäisches Patentamt European Patent Office Office européen des brevets**

(11) **EP 2 431 074 A1**

(12) **EUROPEAN PATENT APPLICATION**

(43) Date of publication:
**21.03.2012 Bulletin 2012/12**

(51) Int Cl.:
**A61N 5/10** $^{(2006.01)}$

(21) Application number: **10177977.5**

(22) Date of filing: **21.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicants:
• **Université Catholique de Louvain**
  **1348 Louvain-La-Neuve (BE)**
• **Atomic Energy Commission of Syria (AECS)**
  **Damascus (SY)**

(72) Inventors:
• **Abboud, Fadi**
  **1083, Bruxelles (BE)**
• **Scalliet, Pierre**
  **1390, Grez-Doiceau (BE)**
• **Vynckier, Stefaan**
  **1560, Hoeilaart (BE)**

(74) Representative: **Pecher, Nicolas et al**
  **Pecher Consultants SPRL**
  **Centre Monnet - Avenue Jean Monnet, 1**
  **1348 Louvain-la-Neuve (BE)**

(54) **System and method for determining radiation dose distribution**

(57)    A system and method are disclosed for determining the radiation dose distribution created by a set of radioactive seeds (20) placed in a volume (30). The method includes three stages. Stage T01 comprises preliminary steps that allow one to obtain the unit radiation dose distribution created by one seed and the individual attenuations created by its neighboring seeds when said neighboring seeds are assumed to be positioned at the nodes of a three-dimensional periodic lattice. Steps of stage T02 load some results of the preliminary steps, said results being used by steps of stage T03 that actually determine the dose distribution created by a set of seeds (20). The invention also relates to an apparatus, a program and a computer readable medium adapted for the execution of this method.

Fig. 9

**Description**

**TECHNICAL FIELD**

The present invention relates to a method, an apparatus, a program and a computer readable medium for determining the radiation dose distribution created by a set of radioactive seeds placed in a volume.

**DESCRIPTION OF RELATED ART**

Brachytherapy represents an effective treatment option for different types of cancer. In contrast to external beam radiotherapy in which high-energy particles are directed at the tumor from outside the body, brachytherapy involves the precise placement of radiation sources directly at the site of the cancerous tumor. As an example, for the treatment of the prostate cancer, $^{125}$I or $^{103}$Pd isotopes are encapsulated in small seeds (typically 5 mm long and 0.5 mm in diameter) that are inserted into the volume to be irradiated.

A critical part of treatment planning is an accurate determination of dose distribution in the patient. Treatment planning systems for prostate brachytherapy with seeds generally use the TG-43 algorithm and its update, TG-43U1 for dose calculations. The TG-43 algorithm is discussed in article by R. Nath et al., entitled "Dosimetry of interstitial brachytherapy sources: Recommandations of the AAPM Radiation Therapy Committee Task Group No. 43", Med. Phys. 22 (2), 209-234 (1995); the TG-43U1 protocol is discussed by M.J. Rivard et al. in "Update of AAPM Task Group No. 43 report: A revised AAPM protocol for brachytherapy dose calculation," Med. Phys. 31 (3), 633-674 (2004).

In TG-43 based dosimetry methods, the dose distributions of the individual seeds are superposed to determine the dose distribution for the whole implant. The calculations of the TG-43 protocol can be made with a treatment planning system at an acceptable speed (less than a second). This allows one to perform the dose optimization in parallel to the insertion of the seeds into the area to be irradiated. Because of tissue heterogeneities and/or positioning errors, some seeds do not locate at their predicted and desired positions. These deviations can be viewed 'on-line', which means during the insertion of the seeds, by using ultrasound-based imaging techniques as an example. A rapid calculation of the dose distribution with these new seed positions is then particularly interesting for placing the following seeds.

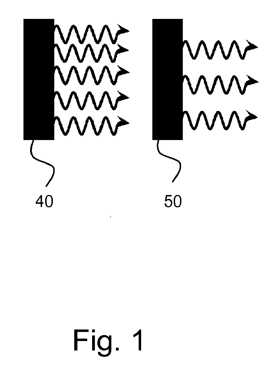

However, one might discuss the accuracy of TG-43 based dosimetry methods. Indeed, one of the assumptions is that the total dose distribution for a multi-seed implant is merely the superposition of the dose distributions due to each seed independently. That means that TG-43 based methods do not take into account the interseed attenuations, known as the interseed effect. The interseed effect is illustrated in figure 1. A seed that is assumed to be non-active, 50, attenuates the radiation dose distribution created by the active seed 40. Studies have shown that the influence of the interseed effect on dose distribution can be large and that it increases with the seeds density. Typically the interseed effect varies between 2 % and 10 %, inducing that the average dose deposited in the prostate is overestimated when using a TG-43 based method. The influence of the interseed effect on dose distribution is notably discussed in the articles by S. G. Burns et al., entitled "The accuracy of single-seed dose superposition for I-125 implants" Med. Phys. 16, 627-631 (1989); J. F. Carrier et al. entitled "Impact of interseed attenuation and tissue composition for permanent prostate implants", Med. Phys. 33 (3), 595-604 (2006); O. Chibani et al., entitled "Dosimetric effect of seed anisotropy and interseed attenuation for 103Pd and 125I prostate implants", Med. Phys. 32(8), 2557-66 (2005).

Full Monte Carlo Simulations (FMCS) techniques directly calculate the total dose of a given seed distribution. One of such methods uses the Monte Carlo N-Particles (MCNP) code (J.F. Briesmeiter, "MCNP general Monte Carlo N-particle transport", Los Alamos National Laboratory, Report No. LA-12625, 2004). These techniques take into account the interseed effect but are very slow because of the large number of seeds (greater than fifty). With computers commonly used in medical applications having a processor such as Intel Core Duo 2.83 GHz, the time of calculation reaches 16 hours per patient. Such a method is discussed in the article by O. Chibani et al., entitled "Dosimetric effect of seed anisotropy and interseed attenuation for 103Pd and 125I prostate implants", Med. Phys. 32(8), 2557-66 (2005). Because of the long calculation time, one cannot use the FMCS method simultaneously with the placement of the seeds.

An accelerated Monte Carlo code, named MCPI, has been developed for dose calculation in prostate brachytherapy (O. Chibani et al., "MCPI: A sub-minute Monte Carlo dose calculation engine for prostate implants", Med. Phys. 32(12), 3688-3698). The MCPI calculation method takes into account the interseed effect and is faster than FMCS techniques. However, the calculation time is about one minute for 83 $^{103}$Pd-based seeds if one uses a single Pentium 4 PC, 2.4 GHz. Moreover, the MCPI technique requires a deep modification of the used Monte Carlo algorithms inducing new complex computational steps.

In response to the concerns discussed above, what is needed is a method, an apparatus, a program or a computer readable medium for quickly determining the radiation dose distribution created by a set of radioactive sources and that takes into account the interseed effect. The word "quickly" means in an acceptable time for the patient and the therapist during the insertion of the seeds, which represents a time of few seconds.

**SUMMARY OF THE INVENTION**

The object of the present invention is to provide a calculation method and a calculation apparatus that quickly determine the dose distribution created by a set of radioactive sources placed in a volume and which take into account the interseed effect, a program for allowing computer to execute the dose calculation method, and a computer readable medium where such program is recorded. The present invention is applicable to many problems in which energy or dose calculations are required.

According to a first aspect of the invention, a method for determining the radiation dose distribution created by a set of radioactive seeds placed in a volume is provided. This method includes three stages, each stage comprising different steps.

The steps of the first stage that are called preliminary steps are: assuming (S01) that seeds are positioned at nodes of a three-dimensional periodic lattice; determining (S02) the unit radiation dose distribution, $D_{Single}(x,y,z)$, created by one seed $SE_0$ when said seed $SE_0$ is alone in the volume and when its centre is positioned at $(x_0,y_0,z_0)$; choosing (S03) in the lattice X neighboring seeds of $SE_0$; choosing (S04) among these X neighboring seeds of $SE_0$, $SE_k$ seeds, $k = 1 \ldots P, P \leq X$; determining (S05) the basic individual attenuations $BIE_k(x,y,z)$, $k = 1 \ldots P$, by subtracting from $D_{single}(x,y,z)$ the radiation dose distribution created by the same seed $SE_0$ in the presence of one of the $SE_k$ seeds that is assumed to be non active; when $P<X$, determining (S06) the individual attenuations $OIE_m(x,y,z)$, m = 1 ... X - P, created by the neighboring seeds of $SE_0$ of step S03 and that are not considered in step S05, from the basic individual attenuations $BIE_k(x,y,z)$ and by applying to them one or more mathematical operations selected from the list of rotation, reflection with respect to a plane or a point.

The second stage is optional and comprises the steps of: loading the unit radiation dose distribution $D_{single}(x,y,z)$ (S07) and the X individual attenuations (S08) determined in steps S05 and S06.

The steps of the last stage are: inputting (S09) the actual positions $(x_n,y_n,z_n)$ of the $S_n$ seeds that are placed in the volume, n = 1 ... $N_s$; determining (S10) the total radiation dose distribution without the interseed effect by adding all the unit radiation dose distributions created by each seed $S_n$, said unit radiation dose distributions being obtained from $D_{single}(x,y,z)$ and to which spatial translations are applied; determining (S11) for each seed the attenuation $IE_n(x,y,z)$ that represents the total attenuation created by the neighboring seeds of each said seed $S_n$, by adding for each said seed $S_n$, the individual attenuations created by its neighboring seeds that are closer from said each seed $S_n$ than one or two predetermined distances, the individual attenuations being obtained from $BIE_k(x,y,z)$ and $OIE_m(x,y,z)$ and to which spatial translations are applied; adding (S12) all the attenuations $IE_n(x,y,z)$, and finally, determining (S13) the radiation dose distribution created by a set of radioactive seeds by subtracting the result of step S12 from the result of step S10.

Preferably, the three-dimensional periodic lattice of step S01 of the method of the invention is a primitive cubic lattice, wherein six neighboring seeds of $SE_0$ are chosen in step S03, these six neighboring seeds being located at the closest neighboring nodes of $SE_0$.

In a more advantageous embodiment, the three-dimensional periodic lattice of step S01 is a primitive cubic or tetragonal lattice, wherein twenty-six neighboring seeds of $SE_0$ are chosen in step S03 and where five seeds are chosen in step S04 among them.

In another embodiment, the three-dimensional periodic lattice of step S01 is a prism with a regular hexagonal base, wherein twenty neighboring seeds of $SE_0$ are chosen in step S03 and where three seeds are chosen in step S04 among them.

The unit radiation dose distribution $D_{single}(x,y,z)$ can be obtained from a calculation using the method of Monte Carlo.

The predetermined distance of step S11 of the method of the invention can be chosen equal to 10 mm for neighboring seeds that do not lie along the same z axis as each said seed $S_n$, and equal to 20 mm for neighboring seeds that lie along the same z axis as each said seed $S_n$, the z axis being parallel to the direction of insertion of the seeds.

According to a second aspect of the invention, an apparatus adapted for calculating the radiation dose distribution created by a set of radioactive seeds placed in a volume is provided. This apparatus comprises: means (M01) for virtually positioning the seeds at nodes of a three-dimensional periodic lattice; means (M02) for determining the unit radiation dose distribution, $D_{single}(x,y,z)$, created by one seed $SE_0$ when said seed $SE_0$ is alone in the volume and when its centre is positioned at $(x_0,y_0,z_0)$; means (M03) for choosing in the lattice X neighboring seeds of $SE_0$; means (M04) for choosing among these X neighboring seeds of $SE_0$, $SE_k$ seeds, k = 1 ... P, P ≤ X; means (M05) for determining the basic individual attenuations $BIE_k(x,y,z)$, k = 1 ... P, by subtracting from $D_{Single}(x,y,z)$ the radiation dose distribution created by the same seed $SE_0$ in the presence of one of the $SE_k$ seeds that is assumed to be non active; means (M06) for determining when $P < X$ the individual attenuations $OIE_m(x,y,z)$, m = 1 ... X - P, created by the neighboring seeds of $SE_0$ chosen by means M03 and that are not considered with means M05 from the basic individual attenuations $BIE_k(x,y,z)$ and by applying to them one or more mathematical operations selected from the list of rotation, reflection with respect to a plane or a point; optional means (M07) for loading the unit radiation dose distribution $D_{Single}(x,y,z)$; optional means (M08) for loading the X individual attenuations determined by means M05 and M06; means (M09) for inputting the actual positions $(x_n,y_n,z_n)$ of the $S_n$ seeds that are placed in the volume (30), n = 1...$N_s$; means (M10) for determining the total radiation dose distribution without the interseed effect by adding all the unit radiation dose distributions created by each seed $S_n$, said unit radiation dose distributions being obtained from $D_{single}(x,y,z)$ and to which spatial translations are applied; means (M11) for determining for each seed the attenuation $IE_n (x, y, z)$ that represents the total attenuation created by the neighboring seeds of each said seed $S_n$, by adding for each said seed $S_n$, the individual attenuations created by its neighboring seeds that are closer from said each seed $S_n$ than one or two predetermined distances, the individual attenuations being obtained from $BIE_k (x,y,z)$ and $OIE_m (x, y, z)$ and to which spatial translations are applied; means (M12) for adding all the attenuations $IE_n (x,y,z)$; means (M13) for determining the radiation dose distribution created by a set of seed by subtracting the result obtained by means M12 from the result obtained by means M10.

An example of three-dimensional periodic lattice used by means M01 is a primitive cubic lattice. In such a case, six neighboring seeds of $SE_0$ can be chosen by means M03, these six neighboring seeds being located at the closest neighboring nodes of $SE_0$. In another embodiment using a primitive cubic lattice or a tetragonal lattice, twenty-six neighboring seeds of $SE_0$ are chosen by means M03, and five seeds are chosen by means M04 among these twenty-six neighboring seeds. A last example of lattice used by means M01 is a prism with a regular hexagonal base. Then, twenty neighboring seeds can be chosen by means M03 and three seeds among them can be chosen by means M04 as an example.

The unit radiation dose distribution $D_{single} (x,y,z)$ can be determined by a calculation unit that uses the method of Monte Carlo.

The predetermined distance used with means M11 can be chosen equal to 10 mm for neighboring seeds that do not lie along the same z axis as each said seed $S_n$, and equal to 20 mm for neighboring seeds that lie along the same z axis as each said seed $S_n$, the z axis being parallel to the direction of insertion of the seeds.

According to a third aspect of the invention, a program for allowing a computer to execute radiation dose calculation processing for determining the radiation dose distribution created by a set of radioactive sources placed in a volume is proposed, said program comprising a code for executing any of the methods described above.

According to a last aspect of the invention, a computer readable medium is provided having stored thereon a program for performing any of the above methods.

**BRIEF DESCRIPTION OF THE DRAWINGS**

Fig.1 is a representation of the interseed effect.

Fig.2 shows a two-dimensional representation of a set of radioactive seeds in a volume.

Fig.3 shows a sketch of a template that is used for the insertion of radioactive seeds for treating the prostate cancer.

Fig.4 shows a cross-section parallel to $\bar{z}$ of a primitive cubic lattice, some nodes of the lattice being occupied by a seed.

Fig.5 shows a plane of a primitive cubic or tetragonal lattice, each node being occupied by a seed.

Fig.6 shows three planes of a primitive cubic lattice. A seed is represented alone in its plane and two other planes are shown, one above and one below this seed.

Fig.7 shows a seed with two neighboring seeds along the same $\bar{z}$ axis.

Fig.8 shows a lattice that is a prism with a regular hexagonal base.

Fig.9 shows a block diagram of a computer apparatus for performing the present invention.

Fig.10(a) shows a cross-section of a prostate with iso-dose curves calculated by the Full Monte Carlo method and by a TG-43 based algorithm. Fig.10(b) shows a cross-section of a prostate with iso-dose curves calculated by the Full Monte Carlo method and by the method of the invention.

Figure 11 (a) shows in a plane the absolute value of the difference in percent between the dose distribution calculated by a method that does not take into account the interseed effect (superposition of unit radiation dose distributions) and by the full Monte Carlo technique. Figure 11 (b) shows in a plane the absolute value of the difference in percent between the dose distribution calculated by the method of the invention and by the full Monte Carlo technique.

**DETAILED DESCRIPTION OF THE INVENTION**

Explanation will be given below in detail with reference to the drawings.

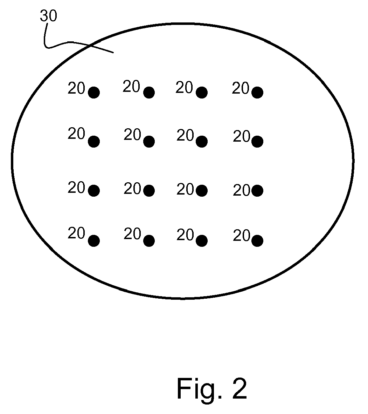

Figure 2 shows an exemplary two-dimensional representation of a set of radioactive seeds 20 that are placed in a volume 30. In this figure, the seeds are assumed to form a square lattice. Typically, the volume 30 represents a structure, such as a tumor of a patient cancer, which is to receive a radiation dose. Figure 2 is a two-dimensional representation for simplicity. However, a real target volume 30 is three-dimensional extending in the $\bar{x}$, $\bar{y}$, and $\bar{z}$ directions. Therefore, the present invention is discussed in three-dimensions. When the radiation dose distribution has to be determined in a volume 30, this said volume 30 is often divided in voxels (see for instance EP 1 128 873). Rectangular parallelepipedic voxels are also defined in Computed Tomography (CT) or Magnetic Resonance Imaging (MRI) scans. Then, the radiation dose distribution is a discretized function and is represented by a matrix extending in the three spatial dimensions, each element of the matrix being equal to the radiation dose in each voxel. The method of the invention can be used with continuous or discretized radiation dose distributions.

The method of the invention includes three stages, T01, T02, and T03, each stage comprising different steps. The first stage T01 includes preliminary steps that have to be carried out only once and whose results can be used for subsequent dose distribution calculations if two conditions are fulfilled. First, the seeds 20 that are in the volume 30 or that are going to be inserted in the volume 30 must be positioned at the nodes of a periodic lattice or at some of them, this lattice being of the same type as the one used in steps of stage T01. Second, the seeds 20 that are in the volume 30 or that are going to be inserted in the volume 30 must be of the same type as the seeds used in the steps of stage T01. These two conditions are detailed below. The second stage T02 includes steps consisting of loading some results obtained in the preliminary steps of stage T01. The third stage T03 comprises different steps which have to be performed each time that a dose distribution is wanted. The different steps are now described in details.

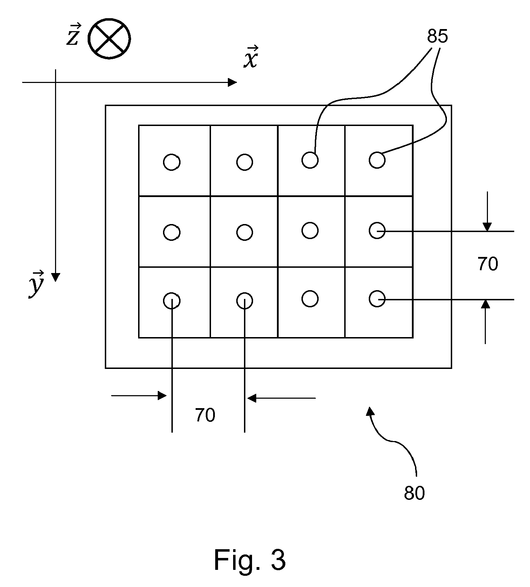

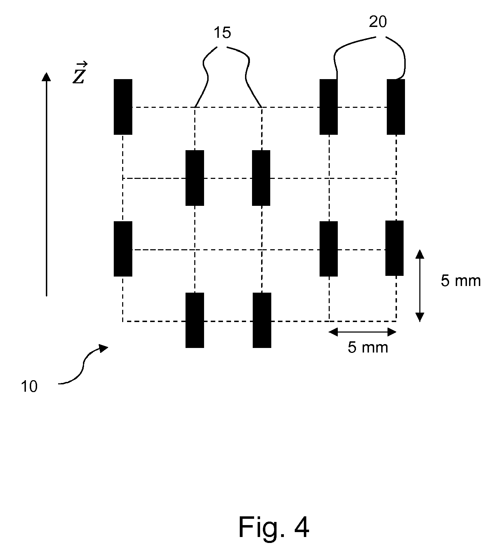

First (step S01), the seeds 20 are assumed to be positioned at the nodes 15 of a three-dimensional periodic lattice 10. Figure 4 shows a cross-section along the z axis of an example of periodic lattice 10. The axis z is typically chosen parallel to the direction of insertion of the seeds. The assumption that seeds are positioned at the nodes of a periodic lattice 10 is made because the seeds are typically inserted following a periodic pattern. Figure 3 shows a sketch of a template 80 that is used for the insertion of radioactive seeds 20 for treating the prostate cancer. Needles carrying the seeds 20 are inserted along the z direction of figure 3, through the holes 85 and into the volume 30 that has to be radiated. The z direction is perpendicular to the plane formed by the holes 85 of the template 80. The distance 70 between each hole along the $\bar{x}$ and $\bar{y}$ directions defined in figure 3 is typically equal to 5 mm. Along the third direction, z, the seeds 20 are typically placed each 5 or 10 mm. As a consequence of the use of the template 80 shown in figure 3, the inserted seeds form a three-dimensional periodic lattice that is a primitive cubic lattice if the seeds are placed each 5 mm along the z direction and a primitive tetragonal lattice if the seeds are positioned each 10 mm along the same z direction. Other templates having a periodic pattern of holes 85 can be used for the insertion of the needles. A template imposing a different spacing of the needles in the $\bar{x}$ and $\bar{y}$ directions results in a positioning of the seeds at the nodes of an orthorhombic lattice whereas a template presenting an hexagonal pattern of holes leads to a positioning of the seeds at the nodes of a prism with a regular hexagonal base. Actually, all the nodes 15 of the lattice 10 are not necessarily occupied by a radioactive seed 20. This is illustrated in figure 4 where each seed is assumed to have the form of a cylinder with an axis parallel to z. In this figure the distance between each node of the lattice is assumed to be equal to 5 mm and only some of them are occupied by a seed 20. In the preliminary steps of the stage T01, the assumption that all the nodes 15 of the lattice 10 are occupied is nevertheless made to deal with a general case whose results will be used partially or totally in stage T03 depending on the actual implementation of the seeds.

To obtain the radiation dose distribution created by a set of radioactive seeds 20, it is necessary to know the unit radiation dose distribution created by one seed that is alone in the volume 30. This is the aim of step S02. The unit radiation dose distribution created by one seed $SE_0$ when said seed is alone and when its centre is assumed to be positioned at $(x_0, y_0, z_0)$ is referred as $D_{single}(x,y,z)$ in the following. As stated above, $D_{single}(x,y,z)$ can be a continuous function or a matrix. As the seed $SE_0$ is assumed to be alone, $D_{single}(x,y,z)$ represents the unit radiation dose distribution without taking into account the attenuation created by the other seeds, i.e. without taking into account the interseed effect. The unit radiation dose distribution $D_{single}(x,y,z)$ can be determined experimentally or from a calculation. In the second case, one can use the method of Monte Carlo with the MCNP5 code as an example. The unit radiation dose distribution $D_{single}(x,y,z)$ is different for each type of radioactive seed. As a consequence, it has to be determined each time that a new type of seed is used. One of the advantages of the invention is that $D_{single}(x,y,z)$ corresponding to one type of seed has to be determined only once. When $D_{single}(x,y,z)$ is saved, it can be used for determining the radiation dose distribution created by different sets of seeds if these seeds are of the same type as the seed used to determine $D_{single}(x,y,z)$. One example of seeds that are used for the treatment of prostate cancers is the seed I25.S06 commercialized by IBt Bebig.

The interseed effect is the attenuation carried out by each seed on the radiation dose distribution created by the other ones. The attenuation carried out by one seed on the radiation dose distribution created by another one decreases as the distance between these two seeds increases. As a consequence, depending on the level of accuracy that is desired, it is not necessary to consider all the neighboring seeds of each seed to evaluate the interseed effect. In step S03, neighboring seeds of $SE_0$ that are used to evaluate the interseed effect around $(x_0, y_0, z_0)$ are chosen. Such neighboring seeds constitute a group of X neighboring seeds of $SE_0$ ($X \geq 1$).

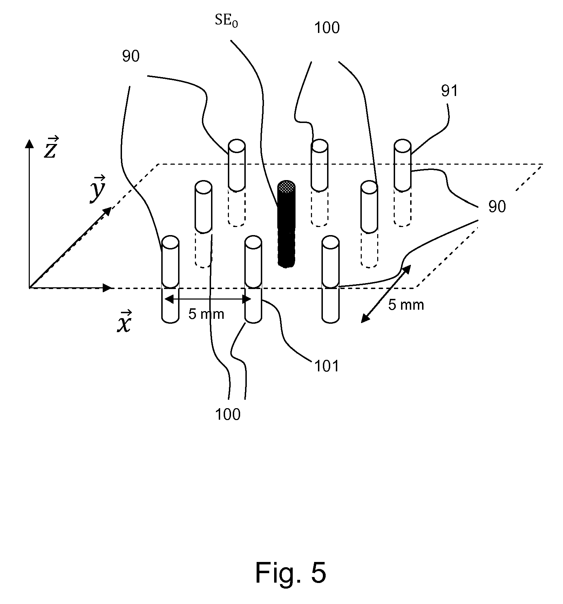

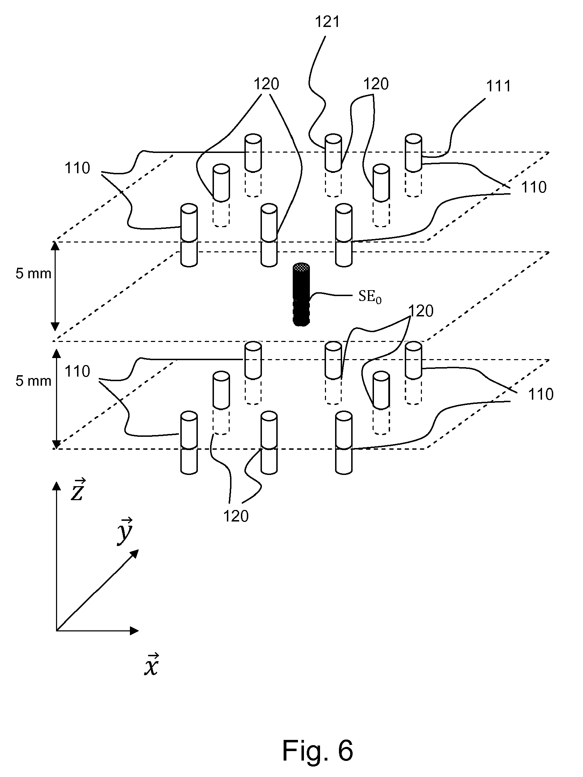

When the lattice 10 is a primitive cubic lattice and when the seeds have a cylindrical shape, one possible choice of the X neighboring seeds of $SE_0$ is shown in figures 5, 6 and 7. Figures 5 and 6 show three planes of such a lattice where the distance between two closest nodes is assumed to be equal to 5 mm. Figure 5 shows a first plane that includes the seed $SE_0$ and where each node of the plane is occupied by a seed 20. Figure 6 shows the planes above and below

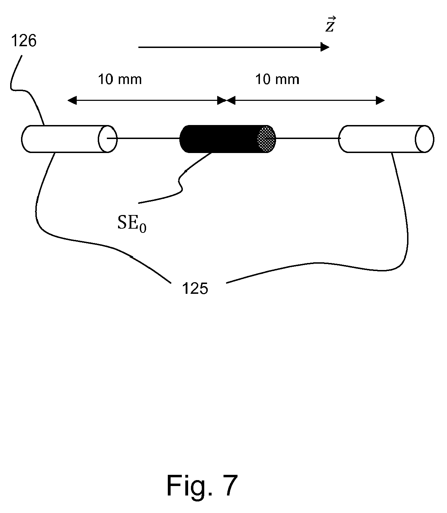

$SE_0$. For clarity reasons, the seeds positioned in the same plane as $SE_0$ are not shown in figure 6. Figure 7 shows the seed $SE_0$ with its two chosen neighboring seeds along the z axis. From these figures, we see that twenty-six (X = 8*3+2 = 26) neighboring seeds of $SE_0$ are chosen. Such a choice results from the evaluation of the importance of the interseed effect by the inventors when I25.S06 seeds are used. For the neighboring seeds that do not lie along the same z axis, the inventors have found that the interseed effect mainly results from the presence of neighboring seeds that are closer than a given distance which is equal to 10 mm for I25.S06 seeds. If one uses the template 80 of figure 3 to insert the seeds 20 in the volume 30, a distance of 10 mm represents two times the distance between two closest nodes of the three-dimensional lattice formed by the seeds 20. So, among the neighboring seeds of $SE_0$ that do not lie along the same z axis, only the seeds such that the distance between their centre and the centre of $SE_0$ is strictly smaller than two times the distance between two closest nodes of the lattice 10 are considered in the calculation of the interseed effect (seeds depicted in figures 5 and 6). Seeds that have a cylindrical shape such as I25.S06 seeds are typically not positioned at all the nodes of the lattice 10 along the z direction. Generally, the distance along z between the centers of such seeds is equal to two times the distance between two closest nodes of the lattice 10. The closest neighboring seeds of $SE_0$ along z are then actually placed at the second neighboring nodes of $SE_0$ along z. The inventors have found that such neighboring seeds significantly contribute to the interseed effect. That is why such seeds are typically chosen to evaluate the interseed effect when I25.S06 seeds are used and when the lattice 10 is a primitive cubic lattice.

If the lattice 10 were a primitive cubic lattice and if spherical seeds were used, one could again choose twenty-six neighboring seeds of $SE_0$ in step S03 but in this case, those along the same z axis as $SE_0$ would be chosen as seeds positioned at the closest nodes of $SE_0$ along z. By using the template of figure 3, this means to choose neighboring seeds along z whose centers are positioned at a distance of 5 mm from the centre of $SE_0$. To decrease the time of calculation, one could rather choose only the closest neighboring seeds of $SE_0$ to evaluate the interseed effect. In the case of a primitive cubic lattice, only six neighboring seeds of $SE_0$ would then be chosen but the precision of the evaluation of the interseed effect would decrease. On the contrary, one could choose more than twenty-six neighboring seeds when the lattice 10 is a primitive cubic lattice. This would result in a larger time of calculation and an increase of precision.

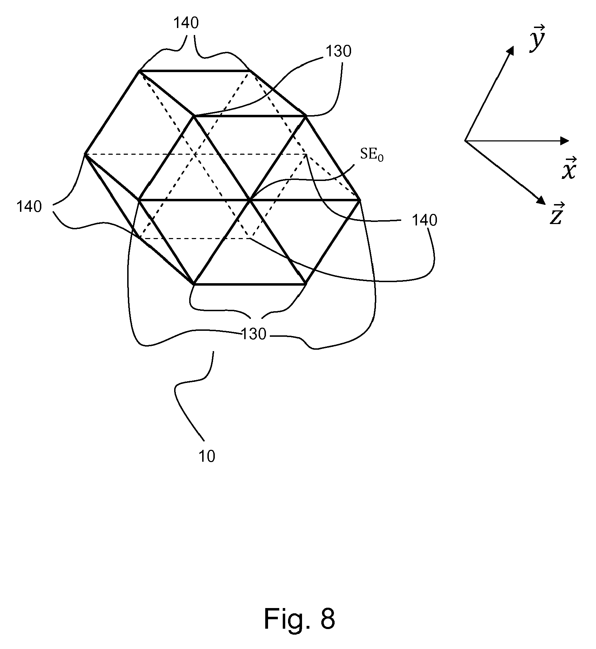

Figure 8 shows another example of a periodic lattice 10 that is a prism with a regular hexagonal base when the distance between each node is equal to 5 mm. Actually, only two planes of such a lattice are shown in figure 8. Each node of this lattice is assumed to be occupied by one cylindrical seed with an axis parallel to z but for clarity reasons, the seeds are not shown in figure 8. One possible choice of the X neighboring seeds of $SE_0$ used to evaluate the interseed effect in this case is to choose twenty seeds, six in the same plan as $SE_0$, six in the upper plane and that do not lie along the same z axis as $SE_0$, six in the lower plane and that do not lie along the same z axis as $SE_0$, and two along the same z axis as $SE_0$ but such that the distance between their center and the center of $SE_0$ is equal to two times the distance between two closest nodes of the lattice (see figure 7).

To determine the attenuation created by the chosen X neighboring seeds of $SE_0$ on its radiation dose distribution, it is not necessary to know the individual attenuations created by each of them. By using the symmetry properties of the lattice 10, one only has to know the individual attenuations created by some of them. From these results, one can deduce the individual attenuations created by the other chosen neighboring seeds by applying to these results mathematical operations that take into account the symmetry of the lattice 10. In steps S04, $SE_k$ seeds are chosen among the X neighboring seeds with $k = 1... P$ and $P \le X$. To decrease the time of calculation, the minimum value of the $P$ parameter could be chosen. This minimum value of $P$ is obtained by choosing among the X neighboring seeds those that produce the same attenuation on the radiation dose distribution created by $SE_0$. We now detail how to determine the minimum value of $P$ when the lattice 10 is a primitive cubic lattice or a prism with a regular hexagonal base. In both cases, we assume that seeds have a cylindrical shape with their axis parallel to z. In the first geometry, we further assume that twenty-six neighboring seeds of $SE_0$ have been chosen in step S03, these neighboring seeds being shown in figures 5, 6 and 7. In the second case, we assume that twenty neighboring seeds have been chosen in step S03: six in the same plane as $SE_0$, six in the upper plane of $SE_0$, six in the lower plane of $SE_0$, and where the distance between these neighboring seeds and $SE_0$ is equal to the distance between two closes nodes of the lattice 10; and finally two seeds along the same z axis as $SE_0$ but such that the distance between their center and the center of $SE_0$ is equal to two times the distance between two closest nodes of the hexagonal lattice of figure 8.

For cylindrical seeds having their axis parallel to z, a primitive cubic lattice as the one shown in figure 5 presents an invariance under rotations of 90° around the z axis. As a consequence, if one knows the attenuation created by one of the seeds 90 of figure 5, one can deduce the individual attenuations created by the other seeds 90. The same property holds for the individual attenuations created by the seeds 100 on the radiation dose distribution created by the seed $SE_0$. For the same reasons, if one knows the attenuation created by one of the seeds 110 of figure 6 on the radiation dose distribution created by the seed $SE_0$, one can deduce the individual attenuations created by the other seeds 110. The same property holds for the individual attenuations created by the seeds 120 of figure 6 on the radiation dose distribution created by the seed $SE_0$. For the two neighboring seeds of $SE_0$ lying along the same z axis (seeds 125 in figure 7), one only has to know the attenuation carried out by one of them. So, one only has to consider one seed among each set of the seeds 90, 100, 110 and 120, and 125, which leads to five seeds. We deduce that for this case, the minimum value of $P$ is equal to five. Step S06 that is detailed below allows one to get the attenuations created by all the chosen neighboring seeds of step S03 from the attenuations created by the $SE_k$ seeds.

We now turn to the hexagonal geometry (figure 8). To obtain the individual attenuations carried out by the twenty chosen neighboring seeds of step S03 on the radiation dose distribution created by $SE_0$, it is only necessary to consider three $SE_k$ seeds among them. Indeed, with respect to the seed $SE_0$, the seeds 130, respectively 140, of figure 8 are equivalent for symmetry reasons. Please note that only half of the seeds 140 is shown in figure 8 for clarity reasons. For the two neighboring seeds of $SE_0$ lying along the same z axis (seeds 125 in figure 7), one only has to keep one of them. If we consider one seed among each set, 125, 130 and 140, one obtains three seeds.

Step S05 aims at determining the individual attenuations carried out by the $SE_k$ seeds on the dose distribution created by the seed $SE_0$. These individual attenuations are named basic individual attenuations $BIE_k(x,y,z)$ (or Basic Interseed Effects), $k = 1 ... P$. To obtain the $BIE_k$ attenuations, one has to subtract from the unit radiation dose distribution, $D_{single}(x,y,z)$, the dose distribution created by the same seed $SE_0$ in the presence of one of the $SE_k$ seeds that is assumed to be non-active. As in step S02, the dose distribution created by the seed $SE_0$ in the presence of one of the $SE_k$ seeds can be determined experimentally or from a calculation. In the first case, one has to measure at different points of the space the radiation dose created by one active seed in the presence of another one that is inactive, the two seeds being placed at the nodes of a periodic lattice. As $D_{single}(x,y,z)$, the attenuations $BIE_k(x,y,z)$ can be continuous functions or matrices.

Step S06 aims at determining the individual attenuations created by the neighboring seeds of $SE_0$, chosen in step S03 and that are not considered in step S05. These individual attenuations are named $OIE_m (x', y', z')$ (or Other Interseed Effects), m = 1 ... $X - P$. Such a step is only necessary when $P < X$. As the seeds are assumed to be positioned at the nodes of a three-dimensional periodic lattice 10, one can obtain these $OIE_m (x',y',z')$ individual attenuations from the basic individual attenuations $BIE_k (x,y,z)$ determined in step S05 and by applying to them mathematical operations which take into account the symmetry of the lattice 10. Seeds typically have the form of a cylinder or of a sphere. In the first case, the axis of the cylinder is typically parallel to the z axis. This leads to two main symmetry properties. Considering a seed $SE_0$ placed in $(x_0,y_0,z_0)$, the first property of symmetry is an invariance of the lattice if it undergoes a rotation of an angle $\theta$ around an axis parallel to the z axis and placed in $(x_0,y_0)$. The value of the $\theta$ angle depends on the type of the periodic lattice 10. For some types of lattices, different $\theta$ angles can be identified. For a primitive cubic lattice, $\theta = 90°$, whereas $\theta = 60°$ for a prism with a regular hexagonal base. The second property of symmetry is an invariance of the lattice if it undergoes a reflection with respect to a plane perpendicular to the z axis and located at $z = z_0$. This type of symmetry is sometimes called mirror symmetry. As a consequence of these symmetry properties, the individual attenuations $OIE_m (x',y',z')$ can be expressed from the basic individual attenuations $BIE_k (x,y,z)$ by using the following equations:

$$OIE_m (x', y', z') = BIE_k(x, y, z), \text{ (Eq 1)}$$

with

$$\begin{pmatrix} x' \\ y' \\ z' \end{pmatrix} = \begin{pmatrix} \cos\theta & -\sin\theta & 0 \\ \sin\theta & \cos\theta & 0 \\ 0 & 0 & 1 \end{pmatrix} \begin{pmatrix} x - x_0 \\ y - y_0 \\ z - z_0 \end{pmatrix} + \begin{pmatrix} x_0 \\ y_0 \\ z_0 \end{pmatrix}, \text{ (Eq 2)}$$

or

$$(x', y', z') = (x, y, 2z_0 - z), \text{ (Eq 3)}$$

or $(x',y',z')$ being determined from a combination of (Eq 2) and (Eq 3). The only condition is that the attenuations $OIE_m$ and $BIE_k$ of equation (Eq 1) correspond to seeds that induce the same attenuation on the radiation dose distribution of $SE_0$. Equation (Eq 2) illustrates the invariance of the lattice if it undergoes a rotation of an angle $\theta$ around an axis parallel to the z axis and placed in $(x_0, y_0)$ whereas equation (Eq 3) is representative of the invariance of the lattice if it undergoes a reflection with respect to a plane perpendicular to the z axis and located at $z = z_0$. In a standard right-handed coordinate system, the angle θ is positive if the rotation is counterclockwise. When the dose distributions are computed in a discretized space, $BIE_k$ and $OIE_m$ are matrices. Some computational systems allow one to perform operations of rotation with matrices. As an example, the software Matlab proposes the function rot90 that allows one to rotate a matrix of 90 ° around an axis. In the case of a primitive cubic lattice with cylindrical seeds, some $OIE_m$ matrices can then be obtained by applying to the corresponding $BIE_k$ matrices this function rot90, eventually several times. Rather than applying a combination of operations of rotation and mirror symmetry, one can apply in an equivalent manner operations of reflection with respect to the node occupied by $SE_0$, $(x_0,y_0,z_0)$, to obtain some attenuations $OIE_m(x',y',z')$ from the basic individual attenuations $BIE_k(x,y,z)$. Then one has to use equation (Eq 1) in combination with the following equation:

$$(x', y', z') = (2x_0 - x, 2y_0 - y, 2z_0 - z) \quad \text{(Eq 4)}$$

To illustrate the operations of step S06, we consider again the case of a primitive cubic lattice such as the one illustrated in figures 5 and 6, with cylindrical seeds. As before, we assume that twenty-six neighboring seeds of $SE_0$ have been chosen in step S03, these twenty-six neighboring seeds being represented in figures 5, 6 and 7. We also assume that among these twenty-six neighboring seeds, five of them have been chosen in step S04 (P = 5). An example for the choice of these five seeds $SE_k$, $k$ = 1 ... 5, is referred in figures 5, 6 and 7 as seeds 91, 101, 111, 121, and 126. If one knows the attenuation due to the seed 91 on the dose distribution created by the seed $SE_0$, one can obtain the individual attenuations carried out by the other seeds referred as 90 in figure 5 by using equation (Eq 1) and successively equation (Eq 2) with θ = 90 °, 180 °, 270 °. The same operations can be applied to the attenuation carried out by the seed 101 to obtain the individual attenuations created by all the seeds 100. We now consider the seeds 111 and 121 of figure 6. If one knows the individual attenuations of such two seeds on the dose distribution created by $SE_0$, one can obtain the individual attenuations carried out by the other seeds 110 and 120 that lie in the same plane by applying the same procedure as the one used when considering the seeds 90 and 100. To obtain the individual attenuations carried out by the seeds 110 and 120 that do not lie in the same plane as 111 and 121, on has to use (Eq 1) and (Eq 3) or (Eq 1) and a combination of (Eq 2) and (Eq 3). In an equivalent manner, one can obtain one individual attenuation carried out by one seed 90 (respectively 100, 110 or 120) by applying to the attenuation due to the seed 91 (respectively 101, 111 or 121) an operation of reflection with respect to the point $(x_0,y_0,z_0)$ by using (Eq 1) in combination with equation (Eq 4). Finally, if one knows the attenuation carried out by the seed 126 of figure 7 on the radiation dose distribution of $SE_0$, one can obtain the attenuation carried out by the other seed 125 by using equation (Eq 1) in combination with equation (Eq 3) or (Eq 4).

Steps S01 to S06 constitute the first stage T01 of the method of the invention. These steps have been discussed by assuming that all the nodes 15 of the lattice 10 are occupied by a seed 20. Depending on the radiation dose that is wanted in the volume 30, some of the nodes 15 of the lattice 10 are occupied by a seed and other ones are empty, leading to different seeds configurations. For a chosen type of lattice and seed, the steps of the stage T01 have to be performed only once if their results are saved, even if all the nodes of the lattice are actually not occupied by a seed. The steps of stage T01 are therefore called preliminary steps.

For a large number of seeds, the method of the invention is preferably performed with a computational system running on a computer. An example of such a system is the software Matlab. If the preliminary steps of stage T01 have been performed prior to the time when the radiation dose distribution is wanted and if the results of the preliminary steps have been saved, one has first to load in the used computational system the unit radiation dose distribution, $D_{single}(x, y,z)$, and the X individual attenuations determined in steps S05 and S06: $BIE_k(x,y,z)$ and $OIE_m(x,y,z)$ that we rename $IE_c(x, y, z)$, $c$ = 1 ... X, where X is the number of neighboring seeds chosen in step S03. Steps S07 and S08 of stage T02 relate to these optional loading phases. These steps have to be performed each time that the used computational system is started. However, once the computational system is running, one does not need to repeat the steps S07 and S08 to obtain the radiation dose distribution created by different seeds configurations if the seeds are positioned at some nodes of a periodic lattice 10 that is always of the same type as the one used to determine the X attenuations of steps S05 and S06.

Steps of the stage T03 have to be performed each time that a radiation dose distribution created by a set of seeds is wanted. First (step S09), the actual positions $(x_n,y_n,z_n)$ of the seeds that are or that will be inserted in the volume 30, are loaded in the computational system. The seeds are named $S_n$, $n$ = 1... $N_s$ and their actual positions can be obtained from a treatment planning and verified by using ultrasound-based imaging techniques as an example.

In step S10, the total radiation dose distribution created by the $N_s$ seeds $S_n$ and without taking into account the interseed effect is calculated. This distribution is called $D_{noIE}(x,y,z)$ and is obtained by adding the individual radiation dose distributions created by each seed. The seeds 20 that are placed in the volume 30 are often of the same type. If this type is the same as the one of the seed that is used in step S02 to determine $D_{single}(x,y,z)$, the individual radiation dose distributions created by each seed are simply equal to this unit radiation dose distribution $D_{single}(x,y,z)$. If the inserted seeds are of different types, it is necessary to determine in step S02 different unit radiation dose distributions corresponding to each type of seeds. In step S02, $D_{single}(x,y,z)$ is determined by assuming that the seed creating this dose is located at $(x_0,y_0,z_0)$. To obtain $D_{noIE}(x,y,z)$, it is necessary to take into account the actual positions $(x_n,y_n,z_n)$ of the $N_s$ seeds $S_n$ when performing the summation of the individual contributions. This is illustrated in the following equation:

$$D_{noIE}(x,y,z) = \sum_{n=1}^{Ns} D_{single}(x + x_0 - x_n, y + y_0 - y_n, z + z_0 - z_n)$$

which summarizes the step S10.

The importance of the attenuation carried out by a neighboring seed on the radiation dose distribution created by another one decreases as the distance between such two seeds increases. For I25.S06 seeds commercialized by IBt Bebig, the inventors have quantified the distance above which the attenuation due to a seed can be neglected in the calculation of the interseed effect. For neighboring seeds that do not lie along the same z axis of a seed (the z axis being parallel to the direction of insertion of seeds and to seed axes), the inventors have found that the interseed effect mainly results from those whose centers are strictly closer than 10 mm from the center of this seed. For neighboring seeds that lie along the same z axis of a seed, the interseed effect mainly results from seeds whose centers are strictly closer than 20 mm from the center of this seed. If another type of seeds were used, this distance criterion could change. In step S11, for each seed $S_n$, the total attenuation created by its neighboring seeds, $IE_n(x,y,z)$, is determined by adding the individual attenuations $IE_c(x, y, z)$ corresponding to neighboring seeds that are located at a distance that is strictly smaller than one or two predetermined criterion's from said each seed $S_n$. The attenuations $IE_c(x,y,z)$ are determined in steps S05, and S06 by assuming that the seed surrounded by neighboring seeds is located at $(x_0,y_0,z_0)$. To obtain $IE_n(x,y,z)$, it is necessary to take into account the actual positions $(x_n,y_n,z_n)$ of the $N_s$ seeds $S_n$ when performing the summation of the individual attenuations. This is illustrated in the following equation:

$$IE_n(x,y,z) = \sum_{c=1} IE_c(x + x_0 - x_n, y + y_0 - y_n, z + z_0 - z_n)$$

To obtain the global interseed effect, $IE(x,y,z)$, one has to add the $N_s$ attenuations determined in step S11. This is the aim of step S12 which is summarized in the following equation:

$$IE(x,y,z) = \sum_{n=1}^{N_s} IE_n(x,y,z)$$

Finally, in step S13, the radiation dose distribution created by the $N_s$ seeds taking into account the interseed effect is obtained by subtracting the global attenuation determined in step S12 from the dose distribution obtained in step S10:

$$D(x,y,z) = D_{noIE}(x,y,z) - IE(x,y,z).$$

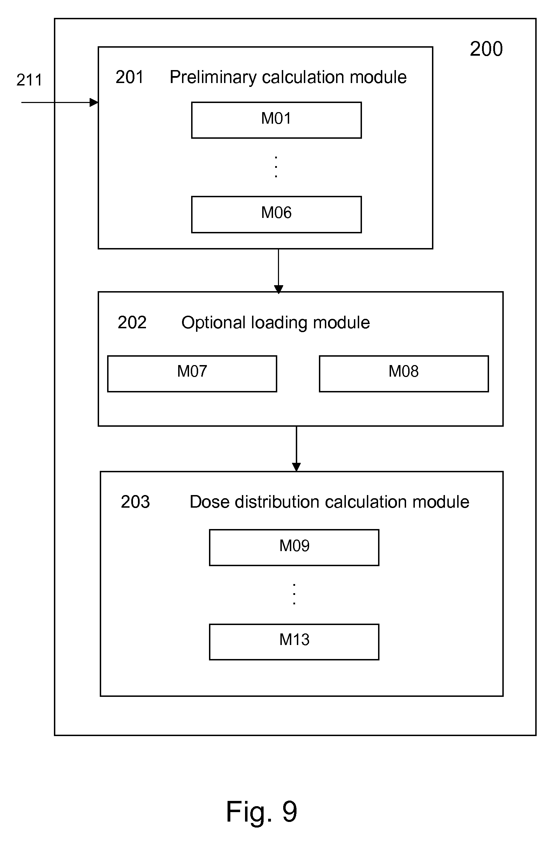

According to one aspect of the invention, the inventors use an apparatus to carry out the method described above. As an example, figure 9 is a block diagram of a computer 200 for performing the present invention. The computer 200 contains three main software modules that implement the various steps of the method of the invention: preliminary calculation module 201, optional loading module 202, and dose distribution calculation module 203. The computer 200 can be an ordinary, single processor personal computer that includes an internal memory for storing computer program instructions which control how a processing unit within the computer accepts, transforms, and outputs data. The internal memory includes both a volatile and a non-volatile portion. Those skilled in the art will recognize that the internal memory can be supplemented with computer memory media, such as a compact disk, flash memory cards, a magnetic disc drive or a dynamic random access memory.

Before the computer 200 can calculate the radiation dose distribution, means M01 (a software component of the module 201) virtually position seeds at the nodes of a three-dimensional periodic lattice 10. Eventually, a user can specify the type of three-dimensional periodic lattice 10 whose nodes are assumed to be occupied by seeds 20, and the type of such seeds 20. Reading means within the preliminary calculation module then accept these input parameters 211 specified by the user. Otherwise, the type of lattice 10 and of seeds 20 is fixed. Four examples of periodic lattice 10 are cubic lattices, tetragonal lattices, orthorhombic lattices, and a prism with a regular hexagonal base. Means M02 (another software component of the module 201) determines the unit radiation dose distribution $D_{single}(x,y,z)$ created by one seed $SE_0$ when said seed $SE_0$ is alone in the volume (30) and when its centre is positioned at $(x_0,y_0,z_0)$. This unit radiation dose distribution $D_{single}(x,y,z)$ can be obtained from a calculation unit that uses the method of Monte Carlo. Means M03 of the module 201 chose in the lattice 10 X neighboring seeds of $SE_0$. When the lattice 10 is a primitive cubic lattice, these X neighboring seeds can be the six closest neighboring seeds of $SE_0$ in said lattice 10. Another choice for such a lattice is to choose twenty-four neighboring seeds as shown in figures 5, 6 and 7. When the lattice 10 is a prism with a regular hexagonal base, twenty neighboring seeds of $SE_0$ can be chosen as shown in figures 7 and 8. Means M04 of the module 201 then chose among these X neighboring seeds of $SE_0$ $SE_k$ seeds with $k = 1 ... P, P \le X$ and means M05 determine the basic individual attenuations $BIE_k(x,y,z)$, $k = 1 ... P$ created by these $SE_k$ seeds. Such a determination is carried out by subtracting from $D_{single}(x,y,z)$ the radiation dose distribution created by the seed $SE_0$ in the presence of one of the seeds $SE_k$ that is assumed to be non active. Typically this operation can be performed by using the method of Monte Carlo. When P < X, means M06 (a last software component of the module 201) determine the individual attenuations $DIE_m(x,y,z)$, $m = 1 ... X - P$, created by the neighboring seeds of $SE_0$ chosen by means M03 and that are not taken into account with means M05, from the basic individual attenuations $BIE_k(x,y,z)$ and by applying to them one or more mathematical operations selected from the list of rotation, reflection with respect to a plane or a point. Software components referred as means M01 to M06 constitute the preliminary calculation module 201. Eventually, an additional means allows one to save the results of this preliminary calculation module 201.

After, the optional loading module 202 comprising the software components referred as means M07 and M08 allows one to load the unit radiation dose distribution $D_{single}(x,y,z)$ and the X individual attenuations determined by means M05 and M06 when these results have been saved.

The dose distribution calculation module 203 that includes components referred as means M09 to M13 actually determines the radiation dose distribution created by a set of radioactive seeds 20 in a volume 30. First, means M09 allows one to input the actual positions $(x_n,y_n,z_n)$ of the $S_n$ seeds that are placed in the volume (30), $n = 1... N_s$. Then, means M10 determine the total radiation dose distribution without the interseed effect by adding all the unit radiation dose distributions created by each seed $S_n$, said unit radiation dose distributions being obtained from $D_{single}(x,y,z)$ and to which spatial translations are applied. For each seed, means M11 determine the attenuation $IE_n(x, y, z)$ that represents the total attenuation created by the neighboring seeds of each said seed $S_n$, by adding for each said seed $S_n$, the individual attenuations created by its neighboring seeds that are closer from said each seed $S_n$ than one or two predetermined distances, the individual attenuations being obtained from means M08 and to which spatial translations are applied. When I25.S06 seeds commercialized by IBt Bebig are used, the inventors have quantified the distance above which the attenuation due to a seed can be neglected in the calculation of the interseed effect. For neighboring seeds that do not lie along the same z axis of a seed, the inventors have found that the interseed effect mainly results from those whose centers are strictly closer than 10 mm from the center of this seed. For neighboring seeds that lie along the same z axis of a seed, the interseed effect mainly results from seeds whose centers are strictly closer than 20 mm from the center of this seed. Means M12 add all the attenuations $IE_n(x, y, z)$ and finally, M13 subtract the result obtained by means M12 from the result obtained by means M10.

The inventors have used a personal computer having the following characteristics, Intel Core Duo 2.83 GHz, and the three modules described above, 201, 202 and 203 to determine the radiation dose distribution created by a set of seeds 20 placed in a volume.

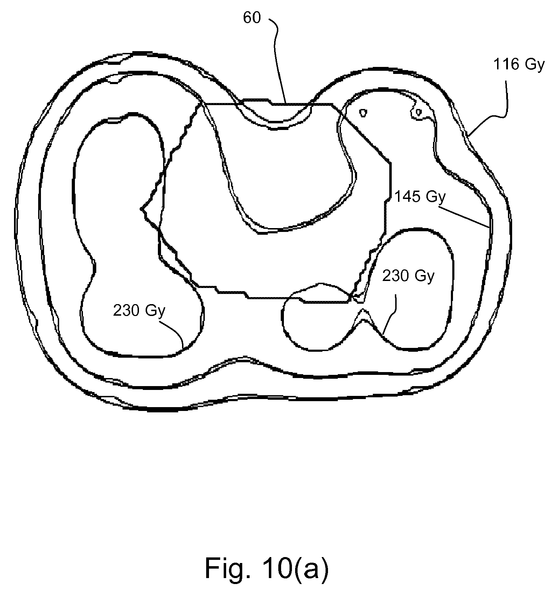

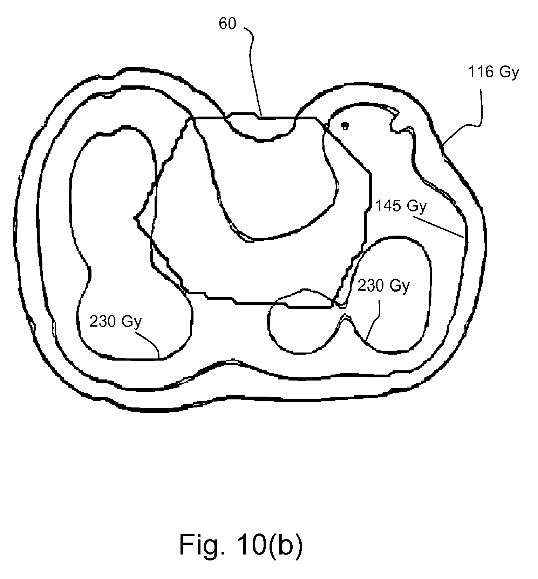

Figures 10 (a) and 10 (b) show two cross-sections of a prostate 60 with iso-dose curves calculated by different methods and corresponding to a given distribution of I25.S06 seeds commercialized by IBt-Bebig. As stated above, a full Monte Carlo calculation for determining the radiation dose distribution created by a set of seeds is long (several hours). Such a method which takes into account the interseed effect can nevertheless be used for benchmarking purposes. Figure 10 (a) shows the iso-dose curves calculated by a TG-43 based method and by the full Monte Carlo method. The iso-dose curves determined by the first method are located at the exterior of those determined by the full Monte Carlo method. A clear difference is observed at the level of the 116 Gy iso-dose curve that is more curved inside the prostate when the interseed effect is taken into account. As a consequence, a TG-43 based method overestimates the dose distribution in the prostate 60. Figure 10 (b) shows the iso-dose curves calculated by the method of the invention and by the full Monte Carlo method. In this figure, the iso-dose curves of both methods are close to each other, indicating the efficiency of the method of the invention to take into account the interseed effect. Actually, a complete comparison between the full Monte Carlo method and the method of the invention for this seeds distribution leads to differences that are at most equal to 3 %, whereas differences up to 10 % are observed between results obtained with the full Monte Carlo technique and the TG-43 based method for the seeds distribution corresponding to figures 10 (a) and 10 (b).

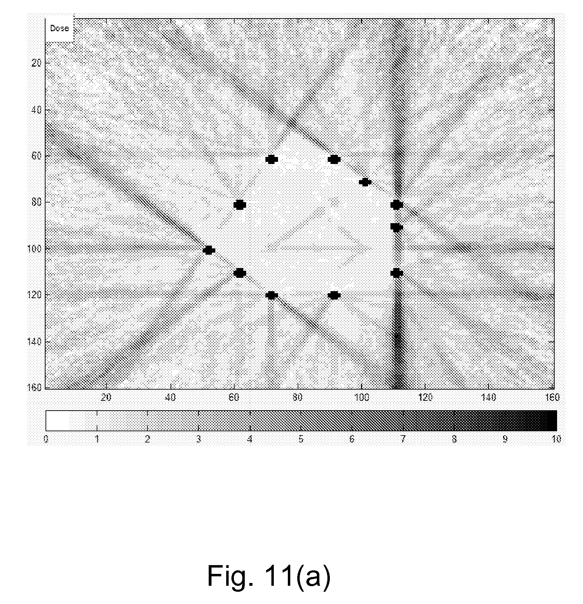

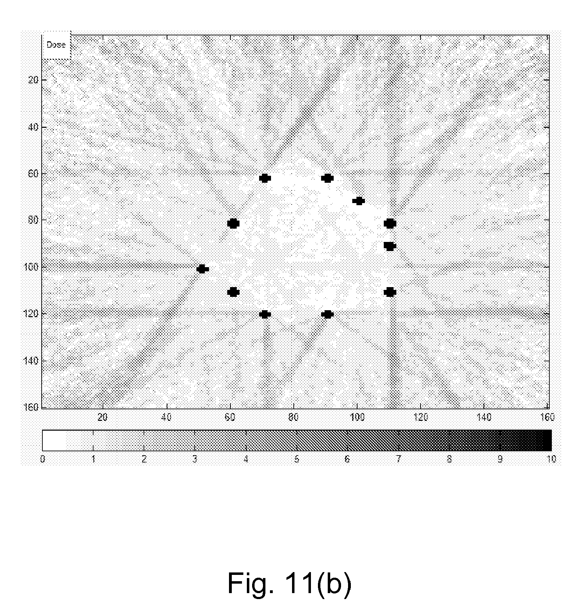

Figure 11 (a) shows in a plane the absolute value of the difference in percent between the dose distribution calculated by a method that does not take into account the interseed effect and by a full Monte Carlo technique. The used method that does not take into account the interseed effect consists in adding the unit radiation dose distributions corresponding to the different seeds, one unit radiation dose distribution being obtained by a Monte Carlo calculation (superposition principle). Figure 11 (b) shows in a plane the absolute value of the difference in percent between the dose distribution calculated by the method of the invention and by a full Monte Carlo technique. Both figures 11 (a) and 11 (b) correspond to the insertion of forty-five I25.S06 seeds. The black dots of figures 11 (a) and 11 (b) represent the seeds that are positioned in the planes of these figures (the scales are in millimeters). The grey levels indicate the absolute value of the differences: white corresponds to 0 % while black corresponds to 10 % or more. By comparing figures 11 (a) and 11 (b), we clearly observe the interseed effect. We also see that the method of the invention corresponds to an increase of precision with respect to a simple superposition technique: for the latter, the difference with respect to the dose distribution calculated by the full Monte-Carlo technique is around 10 %, whereas the same difference is reduced to around 3 % when the method of the invention is used.

Contrary to a full Monte-Carlo calculation method, the method of the invention is fast. For sixty seeds, the steps of the stage T03 (steps S09 to S13) require about 1 second. Due to this fast response, it is possible, using the method and apparatus of the invention to perform a verification of the dose distribution when inserting each of the successive seeds of a set. The method of the invention is also simple: it does not require new complex mathematical steps as it is the case in the MCPI technique.

The terms and descriptions used herein are set forth by way of illustration only and are not meant as limitations. Those skilled in the art will recognize that many variations are possible within the spirit and scope of the invention as defined in the following claims, and their equivalents, in which all terms are to be understood in their broadest possible sense unless otherwise indicated. As a consequence, all modifications and alterations will occur to others upon reading and understanding the previous description of the invention. In particular, dimensions and other parameters, given in the above description may vary depending on the needs of the application.

Summarized, the invention may also be described as follows. A system and method are disclosed for determining the radiation dose distribution created by a set of radioactive seeds (20) placed in a volume (30). The method includes three stages. Stage T01 comprises preliminary steps that allow one to obtain the unit radiation dose distribution created by one seed and the individual attenuations created by its neighboring seeds when said neighboring seeds are assumed to be positioned at the nodes of a three-dimensional periodic lattice. Steps of stage T02 load some results of the preliminary steps, said results being used by steps of stage T03 that actually determine the dose distribution created by a set of seeds (20). The invention also relates to an apparatus, a program and a computer readable medium adapted for the execution of this method.

**Claims**

1. A method for determining the radiation dose distribution created by a set of radioactive seeds (20) placed in a volume (30), including:

    a first stage (T01) comprising the steps of:

        assuming (S01) that seeds are positioned at nodes of a three-dimensional periodic lattice (10);
        determining (S02) the unit radiation dose distribution, $D_{single}(x,y,z)$, created by one seed $SE_0$ when said seed $SE_0$ is alone in the volume (30) and when its centre is positioned at $(x_0,y_0,z_0)$;
        choosing (S03) in the lattice (10) X neighboring seeds of $SE_0$;
        choosing (S04) among these X neighboring seeds of $SE_0$, $SE_k$ seeds, $k = 1 ... P, P \leq X$;
        determining (S05) the basic individual attenuations $BIE_k(x,y,z)$, $k = 1 ... P$, by subtracting from $D_{single}(x,y,z)$ the radiation dose distribution created by the same seed $SE_0$ in the presence of one of the $SE_k$ seeds that is assumed to be non active;
        when $P < X$, determining (S06) the individual attenuations $OIE_m(x,y,z)$, $m = 1 ... X - P$, created by the neighboring seeds of $SE_0$ of step S03 and that are not considered in step S05, from the basic individual attenuations $BIE_k(x,y,z)$ and by applying to them one or more mathematical operations selected from the list of rotation, reflection with respect to a plane or a point;

an optional second stage (T02) comprising the steps of:

loading the unit radiation dose distribution $D_{single}$ $(x,y,z)$ (S07) and the X individual attenuations (S08) determined in steps S05 and S06;

a third stage (T03) comprising the steps of:

inputting (S09) the actual positions $(x_n, y_n, z_n)$ of the $S_n$ seeds that are placed in the volume (30), $n = 1... N_s$; determining (S10) the total radiation dose distribution without the interseed effect by adding all the unit radiation dose distributions created by each seed $S_n$, said unit radiation dose distributions being obtained from $D_{single}(x,y,z)$ and to which spatial translations are applied; determining (S11) for each seed the attenuation $IE_n(x,y,z)$ that represents the total attenuation created by the neighboring seeds of each said seed $S_n$, by adding for each said seed $S_n$, the individual attenuations created by its neighboring seeds that are closer from said each seed $S_n$ than one or two predetermined distances, the individual attenuations being obtained from $BIE_k(x,y,z)$ and $OIE_m(x,y,z)$ and to which spatial translations are applied; adding (S12) all the attenuations $IE_n(x,y,z)$; determining (S13) said radiation dose distribution by subtracting the result of step S12 from the result of step S10.

2. Method according to claim 1, wherein the three-dimensional periodic lattice (10) of step S01 is a primitive cubic lattice and wherein six neighboring seeds of $SE_0$ are chosen in step S03, these six neighboring seeds being located at the closest neighboring nodes of $SE_0$.

3. Method according to claim 1, wherein the three-dimensional periodic lattice (10) of step S01 is a primitive cubic or tetragonal lattice, wherein twenty-six neighboring seeds of $SE_0$ are chosen in step S03 and where five seeds are chosen in step S04 among these twenty-six seeds.

4. Method according to claim 1, wherein the three-dimensional periodic lattice (10) of step S01 is a prism with a regular hexagonal base, wherein twenty neighboring seeds are chosen in step S03 and where three seeds are chosen among these twenty seeds in step S04.

5. Method according to any of the previous claims, wherein the unit radiation dose distribution $D_{single}(x,y,z)$ is obtained from a calculation using the method of Monte Carlo.

6. Method according to any of the previous claims, wherein the predetermined distance of step S11 is equal to 10 mm for neighboring seeds that do not lie along the same z axis as each said seed $S_n$, and equal to 20 mm for neighboring seeds that lie along the same z axis as each said seed $S_n$, the z axis being parallel to the direction of insertion of the seeds.

7. An apparatus adapted for calculating the radiation dose distribution created by a set of radioactive seeds (20) placed in a volume (30), comprising:

means (M01) for virtually positioning the seeds at nodes of a three-dimensional periodic lattice (10); means (M02) for determining the unit radiation dose distribution, $D_{Single}(x,y,z)$, created by one seed $SE_0$ when said seed $SE_0$ is alone in the volume (30) and when its centre is positioned at $(x_0, y_0, z_0)$; means (M03) for choosing in the lattice (10) X neighboring seeds of $SE_0$; means (M04) for choosing among these X neighboring seeds of $SE_0$, $SE_k$ seeds, $k = 1 ... P, P \le X$; means (M05) for determining the basic individual attenuations $BIE_k(x,y,z)$, $k = 1 ... P$, by subtracting from $D_{single}(x,y,z)$ the radiation dose distribution created by the same seed $SE_0$ in the presence of one of the $SE_k$ seeds that is assumed to be non active; means (M06) for determining when $P < X$ the individual attenuations $OIE_m(x,y,z)$, m = 1 ... $X - P$, created by the neighboring seeds of $SE_0$ chosen by means M03 and that are not considered with means M05 from the basic individual attenuations $BIE_k(x,y,z)$ and by applying to them one or more mathematical operations selected from the list of rotation, reflection with respect to a plane or a point; optional means (M07) for loading the unit radiation dose distribution $D_{single}(x,y,z)$ optional means (M08) for loading the X individual attenuations determined by means M05 and M06; means (M09) for inputting the actual positions $(x_n, y_n, z_n)$ of the $S_n$ seeds that are placed in the volume (30), n

= 1 ... $N_s$;

means (M10) for determining the total radiation dose distribution without the interseed effect by adding all the unit radiation dose distributions created by each seed $S_n$, said unit radiation dose distributions being obtained from $D_{single}(x,y,z)$ and to which spatial translations are applied;

means (M11) for determining (S11) for each seed the attenuation $IE_n(x,y,z)$ that represents the total attenuation created by the neighboring seeds of each said seed $S_n$, by adding for each said seed $S_n$, the individual attenuations created by its neighboring seeds that are closer from said each seed $S_n$ than one or two predetermined distances, the individual attenuations being obtained from $BIE_k(x,y,z)$ and $OIE_m(x, y, z)$ and to which spatial translations are applied;

means (M12) for adding all the attenuations $IE_n(x,y,z)$;

means (M13) for determining said radiation dose distribution by subtracting the result obtained by means M12 from the result obtained by means M10.

8. Apparatus according to claim 7 wherein the three-dimensional periodic lattice (10) is a primitive cubic lattice and where six neighboring seeds of $SE_0$ are chosen by means M03, these six neighboring seeds being located at the closest neighboring nodes of $SE_0$.

9. Apparatus according to claim 7 wherein the three-dimensional periodic lattice (10) is a primitive cubic or tetragonal lattice, where twenty-six neighboring seeds of $SE_0$ are chosen by means M03, and where five seeds are chosen by means M04 among these twenty-six seeds.

10. Apparatus according to claim 7 wherein the three-dimensional periodic lattice (10) is a prism with a regular hexagonal base, where twenty neighboring seeds are chosen by means M03 and where three seeds are chosen among these twenty seeds by means M04.

11. Apparatus according to any of claims 7 to 10 wherein the unit radiation dose distribution $D_{single}(x,y,z)$ is determined by a calculation unit that uses the method of Monte Carlo.

12. Apparatus according to any of claims 7 to 11, wherein the predetermined distance used with means M11 is equal to 10 mm for neighboring seeds that do not lie along the same z axis as each said seed $S_n$, and equal to 20 mm for neighboring seeds that lie along the same z axis as each said seed $S_n$, the z axis being parallel to the direction of insertion of the seeds.

13. A program for allowing a computer to execute radiation dose calculation processing for determining the radiation dose distribution created by a set of radioactive sources (20) placed in a volume (30), the program comprising a code for executing the method of any of claims 1 to 6.

14. A computer readable medium adapted so that a program for allowing a computer to execute radiation dose calculation processing for determining the radiation dose distribution created by a set of radioactive sources (20) placed in a volume (30) is recorded, the program comprising a code for executing the method of any of claims 1 to 6.

40   50

Fig. 1

Fig. 2

Fig. 3

Fig. 4

Fig. 5

Fig. 6

Fig. 7

Fig. 8

Fig. 9

Fig. 10(a)

Fig. 10(b)

Fig. 11(a)

Fig. 11(b)

Europäisches
Patentamt

European
Patent Office

Office européen
des brevets

**EUROPEAN SEARCH REPORT**

Application Number

EP 10 17 7977

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAO M., ET AL.: "A Simple Algorithm to Account for Inter-Seed Effects in the Dose Calculation of Prostate Implant Brachytherapy", MEDICAL PHYSICS, vol. 34, no. 6, 6 June 2007 (2007-06-06), page 2410, XP002624536, * the whole document * | 1-14 | INV. A61N5/10 |
| A | RIVARD M.J., ET AL.: "The evolution of brachytherapy treatment planning", MED. PHYS., vol. 36, no. 6, June 2009 (2009-06), pages 2136-2153, XP002624537, * the whole document * | 1-14 | |
| A | ZHOU C., ET AL.: "Distortions induced by radioactive seeds into interstitial brachytherapy dose distributions", MED.PHYS., vol. 31, no. 12, December 2004 (2004-12), pages 3393-3405, XP002624538, * the whole document * | 1-14 | TECHNICAL FIELDS SEARCHED (IPC) A61N |
| A | WO 2010/011844 A1 (TUFTS MEDICAL CT [US]; RIVARD MARK J [US]) 28 January 2010 (2010-01-28) * the whole document * | 1-14 | |

The present search report has been drawn up for all claims

| Place of search | Date of completion of the search | Examiner |
|---|---|---|
| Munich | 1 March 2011 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 17 7977

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2011

| Patent document cited in search report | Publication date | Patent family member(s) | Publication date |
|---|---|---|---|
| WO 2010011844 A1 | 28-01-2010 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1128873 A

**Non-patent literature cited in the description**

- **R. NATH et al.** Dosimetry of interstitial brachytherapy sources: Recommandations of the AAPM Radiation Therapy Committee Task Group No. 43. *Med. Phys.,* 1995, vol. 22 (2), 209-234
- **M.J. RIVARD et al.** Update of AAPM Task Group No. 43 report: A revised AAPM protocol for brachytherapy dose calculation. *Med. Phys.,* 2004, vol. 31 (3), 633-674
- **S. G. BURNS et al.** The accuracy of single-seed dose superposition for I-125 implants. *Med. Phys.,* 1989, vol. 16, 627-631
- **J. F. CARRIER et al.** Impact of interseed attenuation and tissue composition for permanent prostate implants. *Med. Phys.,* 2006, vol. 33 (3), 595-604
- **O. CHIBANI et al.** Dosimetric effect of seed anisotropy and interseed attenuation for Pd and I prostate implants. *Med. Phys.,* 2005, vol. 32 (8), 2557-66
- **J.F. BRIESMEITER.** MCNP general Monte Carlo N-particle transport. Los Alamos National Laboratory, 2004
- **O. CHIBANI et al.** MCPI: A sub-minute Monte Carlo dose calculation engine for prostate implants. *Med. Phys.,* vol. 32 (12), 3688-3698